# EUROPEAN PATENT SPECIFICATION

(11) **EP 0 602 688 B1**
(45) Date of publication and mention of the grant of the patent: **19.06.2002**
(21) Application number: 93121015.7
(22) Date of filing: 01.02.1991
(51) Int. Cl.: C12N 15/62, C12N 1/21, A61K 38/19, C12N 15/57

(54) **Methionine free G-CSf from prokaryotes**
Methioninfreier G-CSF aus Prokaryonten
G-CSF (Facteur de stimulation de colonie de granulocytes) produits par procamyotes et sans méthionire N-terminale

(30) Priority: 03.02.1990 DE 4003149; 17.05.1990 DE 4015921; 17.05.1990 DE 4015922; 10.12.1990 DE 4039415
(43) Date of publication of application: 22.06.1994
(62) Divisional of application: 91902956.1
(73) Proprietor: Max-Planck-Gesellschaft zur Förderung der Wissenschaften e.V., D-37073 Göttingen (DE)
(72) Inventor: Schumacher, Günter, Dr., D-82347 Bernried (DE); Dony, Carola, Dr., D-82319 Starnberg (DE); Meyer, Thomas F., D-72076 Tübingen (DE); Pohlner, Johannes, D-72074Tübingen (DE)
(74) Representative: Huber, Bernhard, Dipl.-Chem.

(56) References cited:
- EP-A- 0 215 126
- EP-A- 0 220 520
- EP-A- 0 256 843
- EP-A- 0 344 796
- GENE. vol. 65 , 1988 , AMSTERDAM NL pages 13 - 22 DEVLIN, P.E. ET AL.; 'Alteration of amino-terminal codons of human granulocyte-colony-stimulating factor increases expression levels and allows efficient processing by methionine aminopeptidase in Esherichia Coli'

## Description

Die Erfindung betrifft ein Verfahren zur sequenzspezifischen Spaltung von biotechnologisch gewonnenen Proteinen unter Verwendung von IgA-Proteasen (auch als Igasen bezeichnet) und insbesondere ein· Verfahren zur Herstellung rekombinanter Proteine bzw. Peptide in Prokaryonten und die anschließende Entfernung einer N-terminalen Sequenz.

Die biotechnologische Darstellung von Proteinen wird vorzugsweise unter Verwendung von Mikroorganismen durchgeführt, die sich leicht züchten lassen und die Gewinnung des erzeugten Proteins auf einfache Weise gestatten. Geeignete Mikroorganismen hierfür sind z.B. das gramnegative Bakterium Escherichia coli, das grampositive Bakterium Streptococcus carnosus sowie die Bäckerhefe Saccharomyces cerevisiae. Die Expression authentischer fremder Gene in solchen Mikroorganismen hat jedoch häufig Nachteile. In E.coli z.B. wird der translationsbedingte aminoterminale Methioninrest natürlicher Proteine von Proteasen meist effizient abgespalten. Bei fremden Proteinen geschieht diese Abspaltung des ersten Methioninrestes jedoch meist nur partiell. Ein geeigneter Weg, solche Proteine mit einem definierten Aminoende herzustellen, ist es daher, diese zunächst in Form von fusionierten Proteinen zu bilden und im Anschluß mit einer sequenzspezifischen Protease definiert zu spalten.

Gegenüber authentischen Proteinen können solche fusionierten Proteine darüber hinaus den Vorzug besitzen, in der Zelle des Mikroorganismus zu aggregieren und dichte Präzipitationskörper ("Inclusion Bodies") zu bilden, die mit geringem Aufwand von anderen Zellteilen abtrennbar sind und somit die Isolierung und Reinigung des gewünschten Proteins erleichtern. Andererseits verleihen Trägerproteine, die mittels gentechnischer Verfahren zunächst an ein eigentlich erwünschtes Protein fusioniert werden, dem Fusionspartner besondere Stabilität gegen unspezifischen proteolytischen Abbau; das Problem des Abbaus von Polypeptiden, die als fremd erkannt werden, betrifft insbesondere die biotechnologische Darstellung kleiner Peptide. Wiederum andere Trägerproteine erlauben, die gewünschten Proteine in bestimmte Zellkompartimente zu steuern, aus welchen sie besonders leicht aufgereinigt werden können, wo sie besonders stabil sind und/oder wo sie für Testzwecke zugänglich sind. Schließlich können Trägerproteine auch spezielle Eigenschaften besitzen, die eine effiziente Aufreinigung, z.B. durch Affinitätschromatographie, erlauben. Für die meisten Anwendungszwecke sind fusionierte Proteine bevorzugt, die das Trägerprotein am Aminoende und das erwünschte Protein am Carboxylende tragen. Aber auch die umgekehrte Version oder die Verknüpfung des erwünschten Proteins mit zwei Fusionspartnern kann in bestimmten Fällen erwünscht sein. Weiterhin kann die Reiteration des erwünschten Proteins innerhalb einer Fusion vorteilhaft sein.

Für die Darstellung des erwünschten Proteins in freier Form aus einer derartigen Fusion ist die Spaltung der kovalent verbundenen Fusionspartner voneinander notwendig. Prinzipiell kann dies mit chemischen oder biochemischen (enzymatischen) Verfahren erreicht werden. Einschränkend wirkt sich jedoch dabei meist die begrenzte Spezifität der bisher zur Verfügung stehenden Verfahren aus; denn es ist wichtig, um das erwünschte Protein zu erhalten, daß eine derartige Spaltung in einer Spaltsequenz zwischen den Fusionspartnern, also der Übergangsregion, aber keinesfalls zusätzlich innerhalb des erwünschten Proteins selbst, erfolgt. Die Spaltung der Fusionspartner muß daher hochspezifisch erfolgen.

Bisher verwendete chemische Verfahren für die sequenzspezifische Trennung von fusionierten Proteinen sind beispielsweise die Spaltung durch Bromcyan an der Aminosäure Methionin innerhalb eines Proteins und die Spaltung zwischen den Aminosäuren Asp · ! · Pro im sauren Milieu unter Verwendung von Ameisensäure. Diese Verfahren sind aber nur dann geeignet, wenn die spezifische Spaltstelle in dem erwünschten Protein, abgesehen von der Übergangsregion zum Fusionspartner, kein weiteres Mal vorhanden ist. Allgemein sind jedoch biochemische Spaltverfahren chemischen Verfahren vorzuziehen, weil erstere meist unter physiologischen oder zumindest chemisch milden Reaktionsbedingungen durchgeführt werden können, die das erwünschte Protein nicht schädigen.

Biochemische Verfahren zur Spaltung von fusionierten Proteinen basieren auf der Verwendung möglichst spezifischer Proteasen. Beispielsweise spaltet Trypsin die auf die Aminosäuren Arginin oder Lysin folgenden Peptidbindungen innerhalb von Proteinen. Eine Erhöhung der Spezifität kann durch vorangehende chemische Modifikation der Aminosäure Lys erzielt werden, wodurch sich die spezifische Erkennung auf die Aminosäure Arginin reduzieren läßt. Eine weitere biotechnologisch verwendete Protease ist Clostripain; dieses Enzym spaltet Peptidbindungen zwischen der Aminosäure Arginin und einer beliebigen darauffolgenden Aminosäure. Eine Übersicht über bisher eingesetzte enzymatische Verfahren zur Spaltung von fusionierten Proteinen wurde von F.A.O. Marston (In [D.M. Glover, E.]: DNA cloning III, IRL PRESS Oxford und Washington DC, 1987) verfaßt. Auch enzymatische Spaltverfahren werden dadurch eingeschränkt, daß die für die Spaltstelle spezifische(n) Aminosäure(n) zugleich auch in dem erwünschten Protein selbst vorkommen können. Zur biochemischen Spaltung von Proteinfusionen eignen sich daher besonders Enzyme, die zur Spaltung nicht nur eine Aminosäure, sondern eine Abfolge von Aminosäuren erkennen; denn die Wahrscheinlichkeit, daß eine bestimmte Aminosäureabfolge außer in der Spaltstelle zwischen den Fusionspartnern in dem erwünschten Protein selbst noch einmal vorkommt, ist umso geringer, je größer die Anzahl der für die Erkennung und Spaltung einer Spaltsequenz notwendigen Aminosäuren ist.

Proteasen, die ein bestimmtes Protein sehr spezifisch schneiden, sind bekannt. Die Mehrzahl solcher selektiver Proteasen (die z.B. im Komplement- und Blutgerinnungssystem des Menschen vorkommen) spaltet zwar an einer definierten Stelle des Substrats; bei Übertragung der entsprechenden Spaltregion in ein anderes Protein (z.B. ein fusioniertes Protein) sind derartige Proteasen im Regelfall jedoch nicht mehr in der Lage, zu spalten. Die Gründe hierfür sind vielfältig und liegen beispielsweise in der Erkennung einer bestimmten Sekundär- oder Tertiär-Struktur im Substrat durch die Protease oder in der Unzugänglichkeit der Spaltstelle in fusionierten Proteinen.

Die Liste sequenzspezifischer Proteasen, die für die Spaltung von fusionierten Proteinen bisher bedingt eingesetzt werden, wird zur Zeit von dem Faktor Xa angeführt. Diese Protease schneidet spezifisch die Spaltsequenz Ile-Glu-Gly-Arg·!·X wobei ·!· die Spaltstelle und X eine beliebige Aminosäure angibt. Es zeigt sich jedoch, daß auch diese Protease nicht generell zur Spaltung von fusionierten Proteinen, die eine entsprechende Spaltsequenz in ihrer Übergangsregion tragen, einsetzbar ist; häufig werden solche Substrate (d.h. fusionierte Proteine, die ein erwünschtes Protein kovalent gebunden an ein Trägerprotein enthalten) überhaupt nicht, nur zu einem geringen Ausmaß oder nur in löslicher Form gespalten.

Besonders bedeutsam ist eine effiziente Spaltung von fusionierten Proteinen bei der Herstellung von rekombinanten Proteinen in prokaryontischen Organismen. Dort ist nämlich die Klonierung einer DNA-Sequenz erforderlich, die ein AUG als Initiationscodon vor dem Beginn der eigentlichen DNA-Sequenz enthält. Als Folge davon wird in Prokaryonten wie z.B. E. coli ein rekombinantes Protein exprimiert, das einen Methioninrest an Aminosäureposition -1 enthält.

In vielen Fällen ist jedoch eine Bereitstellung von in Position 1 methioninfreien rekombinanten Proteinen erforderlich. Die Gewinnung solcher Proteine aus Prokaryonten kann z.B. über eine Methionin-spezifische Peptidase erfolgen, die das N-terminale Methionin abspaltet. Dieses Verfahren ist jedoch sehr aufwendig, da die Abspaltung nur über Proteinsequenzierung überprüft werden kann. Außerdem ist eine Trennung von in Position -1 Methionin-haltigem und methioninfreiem Protein aufgrund des fast identischen Molekulargewichts sehr schwierig und kann damit nur unvollständig erreicht werden.

Die PCT-Anmeldung WO84/02351 offenbart die Abspaltung N-terminaler Aminosäuren von einem Fusionsprotein. Dabei können mehrere Aminosäuren des Proteins vom N-Terminus her durch schrittweises Abspalten durch eine Exopeptidase, vorzugsweise Leucinpeptidase, bis zu einer Sequenz X-Pro entfernt werden. Die Sequenz X-Pro wird aus diesem Produkt entweder in zwei Schritten oder in einer 1-Schritt-Reaktion unter Verwendung von Postprolin-Dipeptidyl-Aminopeptidase (EC 3.4.14.) abgespalten. Dieses Verfahren besitzt jedoch den Nachteil, daß bedingt durch das schrittweise Abspalten der Aminosäuren vom N-Terminus des Proteins kein einheitliches Produkt, sondern immer ein Produktgemisch entstehen muß, das neben dem gewünschten Produkt sowohl unvollständig als auch zu weit abgebaute Proteine enthält.

Ein weiteres Verfahren zur enzymatischen Spaltung eines Fusionsproteins ist aus dem europäischen Patent Nr. 0 020 290 bekannt. Bei diesem Verfahren wird das Enzym Collagenase zum Abspalten des Fusionsanteils eines Proteins von einer bestimmten Erkennungssequenz verwendet. Danach können weitere Aminosäuren des Fusionsanteils anschließend durch weitere enzymatische Behandlung entfernt werden. Es wurde jedoch festgestellt, daß Collagenase und auch andere Endopeptidasen nur eine geringe Spezifität besitzen (siehe Biochim. Biophys. Acta 271 (1972), 133-144). Überdies sind die Collagenasen nur bei Proteinen aktiv, die eine spezifische räumliche Struktur besitzen.

Auch die Verwendung des bereits erwähnten Faktor Xa zur Abspaltung eines N-terminalen Fusionsanteils von Proteinen ist bekannt. Doch auch dieses Verfahren zeigt neben den bereits erwähnten Problemen der Spaltungseffizienz weitere Nachteile, und zwar in der Art, daß möglicherweise auch interne Sequenzen des Proteins erkannt und gespalten werden. Darüberhinaus muß Faktor Xa aus Rinderserum isoliert werden, wodurch bei der Spaltung therapeutisch verwendeter Proteine anschließend eine aufwendige Reinigung und Analytik erforderlich ist, um eventuell vorhandene Pathogenitätsfaktoren bzw. Viren nachzuweisen.

Verschiedene pathogene Bakterienarten (z.B. der Gattung Neisseria, wie Neisseria gonorrhoea und Neisseria meningititis oder der Gattung Haemophilus wie Haemophilus influenzae und Haemophilus aegypticus), die auf der menschlichen Schleimhaut wachsen, sekretieren untereinander in ihrer Sequenz nahe verwandte Proteasen, die spezifisch für menschliches IgAl sind und daher zusammenfassend als IgA-Proteasen oder Igasen bezeichnet werden. Das Immunglobulin IgA1 ist eine wichtige Komponente der sekretorischen Immunantwort, die gegen Infektionen solcher pathogener Organismen schützen soll (Übersicht: Kornfeld und Plaut, Rev. Infect. Dis. 3 (1981), 521-534). Daneben spaltet die IgA-Protease auch ihr eigenes Vorläuferprotein durch Autoproteolyse. Die Bildung der IgA-Protease aus Neisseria gonorrhoeae MS11 im authentischen Bakterienstamm sowie in gramnegativen Wirtszellen wurde bereits ausführlich beschrieben (DE 36 22 221.6).

Die IgA-Protease spaltet folgende Erkennungssequenzen, wie z.B. bei Pohlner et al., (Nature 325 (1987), 458-462) beschrieben:
1. Pro-Ala-Pro·!·Ser-Pro
2. Pro-Pro·!·Ser-Pro
3. Pro-Pro·!·Ala-Pro
4. Pro-Pro·!·Thr-Pro

Dabei bedeutet das Symbol ·!· jeweils die Schnittstelle durch die IgA-Protease. Die Klonierung der IgA-Protease ist z.B. bei Pohlner et al., 1987, supra beschrieben.

Es war daher Aufgabe der vorliegenden Erfindung, ein verbessertes Verfahren zur biochemischen (enzymatischen) Spaltung von fusionierten Proteinen bereitzustellen, so daß gentechnologisch hergestellte fusionierte Proteine, bestehend aus beliebigen Fusionspartnern und einer spezifischen Spaltsequenz in der Übergangsregion, als Substrat für die Gewinnung erwünschter Proteine mit möglichst hoher Ausbeute und reproduzierbar eingesetzt werden können.

Diese Aufgabe wurde gentechnologisch gelöst durch die Erführung der Erkennungsstelle bzw. Spaltsequenz Pro· ! · X-Pro, in die Übergangsregion von fusionierten Proteinen und durch die spezifische Spaltung dieser Spaltsequenz durch eine IgA-Protease an der mit · ! · bezeichneten Spaltstelle, wobei X vorzugsweise für die Aminosäuren Ser, Thr und Ala und besonders bevorzugt für Ser oder Thr steht, aber auch für andere Aminosäuren stehen kann.

Ein Gegenstand der vorliegenden Erfindung ist daher ein Verfahren zur enzymatischen Spaltung von fusionierten Proteinen und Gewinnung von gewünschten Anteilen dieser fusionierten Proteine, welches dadurch gekennzeichnet ist,
(1) mit gentechnischen Mitteln eine Übergangsregion, in der zwei Anteile des fusionierten Proteins miteinander verbunden sind, so modifiziert, daß in dieser Übergangsregion mindestens eine IgA-Protease-Erkennungsstelle mit der Aminosäuresequenz Y-Pro·!·X-Pro entsteht, wobei X eine beliebige Aminosäure und Y eine oder mehrere beliebige Aminosäuren sein kann,
(2) das aus Schritt (1) resultierende fusionierte Protein durch IgA-Protease an der mit ·!· bezeichneten Position der Erkennungsstelle spaltet und
(3) nach der Spaltung einen oder mehrere erwünschte Anteile des fusionierten Proteins gewinnt.

Unter den Begriff "IgA-Protease" gemäß vorliegender Erfindung fallen Proteasen, welche spezifisch IgA spalten, die beispielsweise in Rev. Infekt. Dis.3 (1981) 521-534 beschrieben sind. Ebenso geeignet sind aber auch rekombinante IgA-Proteasen, wie sie beispielsweise in DE-A 36 22 221, Proc. Natl. Acad. Sci. USA 79 (1982) 7881-7885, Proc. Natl. Acad. Sci. USA 80 (1983) 2681-2685, Nature 325 (1987) 458-462 und EMBO Jour. 3 (1984) 1595-1601 beschrieben sind.

Bei dem erfindungsgemäßen Verfahren erfolgt die Modifizierung einer Übergangsregion von fusionierten Proteinen vorzugsweise derart, daß in die Übergangsregion eines fusionierten Proteins Nukleotidsequenzen eingebaut werden, die für eine IgA-Protease-Erkennungsstelle oder einen Teil davon kodieren, wobei diese Nukleotidsequenzen vor oder/und hinter einen oder mehrere, für erwünschte Anteile der Proteinfusion kodierenden DNA-Abschnitte eingebaut werden. Vorzugsweise verwendet man zu diesem Zweck Nukleotidabfolgen, die auf chemischem Weg synthetisiert worden sind.

Überraschenderweise wurde festgestellt, daß das erfindungsgemäße Verfahren insbesondere auch zur Spaltung von fusionierten Proteinen geeignet ist, die ursprünglich (d.h. vor der Modifikation der Übergangsregion) keine natürliche IgA-Protease-Erkennungsstelle aufweisen.

Die IgA-Protease-Erkennungsstelle für das erfindungsgemäße Verfahren weist die Aminosäure-Konsensussequenz Y-Pro·!· X-Pro auf. Dabei kann X eine beliebige Aminosäure und Y eine oder mehrere beliebige Aminosäuren bedeuten. Vorzugsweise bedeutet X Serin, Threonin oder Alanin, besonders bevorzugt Serin oder Threonin. Y steht vorzugsweise für mehrere Aminosäuren, die mit der Sequenz Pro, Pro-Ala, Arg-Pro, Pro-Arg-Pro, Ala-Pro-Arg-Pro oder Pro-Ala-Pro-Arg-Pro enden.

Das erfindungsgemäße Verfahren beinhaltet daher, daß eine IgA-Protease-Erkennungsstelle mit mindestens der Konsensus-Spaltsequenz Pro·!·X-Pro in die Übergangsregion eines beliebigen fusionierten Proteins, z.B. zwischen Trägerprotein und dem erwünschtem Protein eingeführt und zur Abspaltung und Gewinnung des erwünschten Proteins durch IgA-Protease benutzt werden kann. Dabei werden in der Spaltsequenz Pro·!· X-Pro vorzugsweise die Aminosäuren Ser, Ala oder Thr in der Position X eingesetzt. Zur weiteren Optimierung der Spaltung an der bezeichneten Stelle können der Spaltsequenz weitere spezielle Aminosäuren vorgeschaltet werden, insbesondere die Aminosäure Pro.

Besonders bevorzugt sind die Aminosäuresequenzen
a) Pro-Ala-Pro·!·Ser-Pro,
b) Pro-Pro·!·Ser-Pro,
c) Pro-Arg-Pro-Pro·!·Ala-Pro
d) Pro-Pro·!·Thr-Pro,
e) Ala-Pro-Arg-Pro-Pro·!·Thr-Pro oder
f) Pro-Ala-Pro-Arg-Pro-Pro·!· Thr-Pro.

Bei Anwendung des erfindungsgemäßen Verfahrens auf die Spaltung von fusionierten Proteinen, in denen das erwünschte Protein einem Trägerprotein nachgeschaltet ist, entsteht nach Spaltung durch IgA-Protease ein Protein, dessen Aminoterminus durch die Sequenz X-Pro charakterisiert ist. Diese Sequenz kann als Teil des erwünschten Proteins von Vorteil, nachteilig oder aber belanglos sein. Von Vorteil ist diese Sequenz im allgemeinen dann, wenn das gentechnologisch dargestellte erwünschte Protein auch in seiner natürlichen Form die entsprechenden beiden Aminosäuren X-Pro an seinem Aminoterminus enthält. Durch ein aminoterimnales X-Pro charakterisierte Proteine, die biotechnologisch wichtig sind, kommen in der Natur vor.

Das erfindungsgemäße Verfahren besitzt gegenüber allen anderen bekannten Verfahren zur Spaltung von Proteinfusionen die Vorzüge, daß es überraschenderweise universell auf fusionierte Proteine, die in ihrer Übergangsregion die angegebene Spaltsequenz tragen, anwendbar ist und daß es sowohl auf unlösliche, lösliche, membranassoziierte und zellgebundene Proteinfusionen angewendet werden kann. Als besonderen Vorteil bietet das Verfahren darüber hinaus die Möglichkeit, fusionierte Proteine bzw. Proteinfusionen in Form von Präzipitationskörpern zu spalten, derart wie sie in Mikroorganismen anfallen und von wo sie als solche leicht angereichert werden können. Ein weiterer Vorteil des Verfahrens besteht darin, daß das verwendete Spaltenzym, Igase, mit geringem Aufwand aus Kulturmedien von nichtpathogenen Bakterien gewonnen werden kann.

Der Einbau der Spaltsequenz für Igase in die Übergangsregion einer Proteinfusion erfolgt durch gentechnische Mittel. So kann beispielsweise eine Abfolge von Nukleotiden bzw. eine Nukleotidsequenz, die für eine Spaltsequenz oder einen Teil davon kodiert, chemisch synthetisiert und zwischen die DNA-Abschnitte für ein Trägerprotein und ein erwünschtes Protein mittels bekannter gentechnischer Mittel eingebaut werden. Entsprechend kann auch eine natürliche Abfolge von Nukleotiden, die für eine geeignete Spaltsequenz oder einen Teil davon kodiert, eingebaut werden. Das für eine Proteinfusion kodierende Gen wird vorzugsweise unter die Kontrolle von geeigneten (vorzugsweise induzierbaren) Expressionssignalen gestellt, so daß eine den Anforderungen entsprechende Produktion von fusionierten Proteinen ermöglicht wird. Als Wirtszellen für die Produktion von Proteinfusionen können geeignete prokaryontische oder eukaryontische (pflanzliche wie tierische) Zellen verwendet werden; aber auch zellfreie Systeme sind möglich. Die dabei verwendeten Trägerproteine können beliebige Funktionen beinhalten, je nachdem welche Eigenschaften sie einer Proteinfusion verleihen sollen wie bestimmte Transportfunktionen, Funktionen, die die Aufreinigung der Proteinfusion oder deren Stabilität verbessern und vieles mehr. Bevorzugte Trägerproteine sind weiter unten erläutert.

Die dem erfindungsgemäßen Verfahren entsprechende Spaltung von Proteinfusionen erfolgt bevorzugt mit Igase, die von einem überproduzierenden nicht-pathogenen Bakterienstamm gebildet und durch Reinigung aus Kulturüberständen gewonnen wird (siehe z.B. DE-36 22 221).

Das erfindungsgemäße Verfahren kann für präparative wie auch für analytische Zwecke eingesetzt werden. Bei präparativem Einsatz dient das Verfahren der biotechnologischen Gewinnung wichtiger Proteine, die z.B. in der Medizin, in der Forschung, im Umweltschutz oder bei industriellen Prozessen bzw. Produkten Verwendung finden können. Bei analytischem Einsatz kann das Verfahren beispielsweise in Verbindung mit geeigneten Expressionssystemen zur Routineuntersuchung von Genfusionen dienen.

Eine bevorzugte Ausführungsform des erfindungsgemäßen Verfahrens zur enzymatischen Spaltung von fusionierten Proteinen und Gewinnung von erwünschten Anteilen dieser fusionierten Proteine ist dadurch gekennzeichnet, daß man
(1) eine Zelle mit einer rekombinanten DNA oder einem rekombinanten Vektor transformiert, wobei die DNA bzw. der Vektor mindestens eine Kopie eines Gens enthält, das für ein fusioniertes Protein kodiert, das mindestens eine IgA-Protease-Erkennungsstelle in einer Übergangsregion enthält,
(2) die transformierte Zelle in einem geeigneten Medium kultiviert,
(3) das für das fusionierte Protein kodierende Gen in der transformierten Zelle zur Expression bringt,
(4) das fusionierte Protein mit IgA-Protease spaltet und
(5) einen oder mehrere erwünschte Anteile des fusionierten Proteins isoliert.

Dabei kann die Behandlung des fusionierten Proteins mit IgA-Protease im Medium (Kulturbrühe), nach dem Zellaufschluß oder/und nach teilweiser oder vollständiger Abtrennung von zellulären Proteinen erfolgen.

Zur Behandlung eines fusionierten Proteins, vorzugsweise eines prokaryontischen Expressionsprodukts, ist es weiterhin bevorzugt, die IgA-Protease in an sich dem Fachmann bekannter Weise, beispielsweise wie in EP-B 0 141 223 oder EP-B 0 141 224 beschrieben, zu immobilisieren.

Eine besonders bevorzugte Anwendung des erfindungsgemäßen Verfahrens ist die Herstellung rekombinanter Proteine bzw. Peptide ohne N-terminalen Methioninrest aus fusionierten Proteinen bzw. Peptiden mit der Aminosäuresequenz Met-Y-Pro · ! · X-Pro-A, worin X für eine beliebige Aminosäure, vorzugsweise für Thr, Ala oder Ser steht, Y eine oder mehrere beliebige Aminosäuren bedeutet, die vorzugsweise, wenn X für Thr oder Ala steht, mit Pro endet oder, wenn X für Ser steht, mit der Sequenz Pro-Ala oder mit Pro-Pro endet, und A eine beliebige Aminosäuresequenz bedeutet. Dabei spaltet man das fusionierte Protein bzw. Peptid mit IgA-Protease und erhält ein Spaltprodukt mit der Aminosäuresequenz X-Pro-A. Beispielsweise beinhaltet dieses Verfahren zur Herstellung rekombinanter Proteine aus prokaryontischen Zellen ohne N-terminalen Methioninrest die folgenden Schritte:
(1) Transformieren einer prokaryontischen Zelle mit einem Gen, das für ein Protein bzw. Peptid mit der Aminosäuresequenz Met-Y-Pro·!·X-Pro-A kodiert, worin X, Y und A die obengenannten Bedeutungen besitzen,
(2) Kultivieren der transformierten Zelle in einem geeigneten Medium und Exprimieren des transformierten Gens,
(3) Spalten des Expressionsprodukts aus der transformierten Zelle mit der Aminosäuresequenz Met-Y-Pro·!· X-Pro-A durch IgA-Protease und
(4) Isolieren des resultierenden Spaltprodukts mit der Aminosäuresequenz X-Pro-A ohne N-terminalen Methioninrest.

Durch das erfindungsgemäße Verfahren lassen sich mit überraschend hoher Ausbeute und guter Spezifität in einem Schritt Proteine ohne N-terminalen Methioninrest gewinnen, welche die N-terminale Sequenz X-Pro besitzen, wobei X vorzugsweise Thr, Ala oder Ser bedeutet.

Der Trägeranteil Y des fusionierten Proteins bedeutet eine Aminosäuresequenz mit mindestens 1, vorzugsweise bis 100, besonders bevorzugt 1 bis 50 Aminosäuren, die mit einer von der IgA-Protease erkannten Spaltungssequenz endet. Steht X für die Aminosäure Serin, so endet Y vorzugsweise mit der Sequenz Pro-Ala oder Pro. Steht X für Thr oder Ala, so endet Y vorzugsweise mit Pro, mehr bevorzugt mit Arg-Pro, Pro-Arg-Pro oder Ala-Pro-Arg-Pro.

In einer besonders bevorzugten Ausführungsform steht Y für mindestens 5 Aminosäuren, die mit der Sequenz Pro-Ala- Pro-Arg-Pro enden. Für das erfindungsgemäße Verfahren sind jedoch an sich alle von der IgA-Protease erkannten Spaltstellen geeignet.

Der Trägeranteil Y kann daneben noch weitere an sich beliebige Aminosäuren, vorzugsweise bis 100, besonders bevorzugt bis 50 Aminosäuren enthalten. Vorzugsweise werden jedoch hierfür solche Aminosäuresequenzen verwendet, die auf DNA-Ebene die Expression des Proteins Met-Y-Pro · ! · X-Pro-A verbessern oder/und auf Aminosäureebene seine Aufreinigung aus der Zelle erleichtern.

Die Expression des Proteins Met-Y-Pro·!· X-Pro-A auf DNA-Ebene kann beispielsweise durch Fusion mit Fragmenten des β-Galactosidasegens verbessert werden, d.h. der Trägeranteil Y umfaßt einen Teil des β-Galactosidase-Proteins. Andere Möglichkeiten, die Expression des Proteins Met-Y-Pro·!· X-Pro-A zu erhöhen, sind dem Fachmann bekannt. Durch Fusion mit anderen Polypeptiden, insbesondere mit hochgeladenen (z.B. poly(Lys, Arg)) oder an bestimmte Substanzen mit hoher Affinität bindenden (z.B. Streptavidin) Polypeptiden bzw. Proteinen kann die Reinigung und Abtrennung des Expressionsprodukts erleichtert werden (siehe z.B. EP-A 0 089 626, EP-A 0 306 610).

Weiterhin ein Gegenstand der vorliegenden Erfindung ist ein fusioniertes Protein, das mehrere Polypeptidanteile enthält und das eingebaut in mindestens einer Übergangsregion zwischen verschiedenen Polypeptidanteilen eine oder mehrere IgA-Protease-Erkennungsstellen mit der Aminosäuresequenz Pro·!·X-Pro aufweist, wobei X eine beliebige Aminosäure, vorzugsweise jedoch Ser, Thr oder Ala bedeutet. Besonders bevorzugt besitzt die Erkennungsstelle die Aminosäuresequenz (a) Pro-Ala-Pro·!·Ser-Pro, (b) Pro-Pro·!·Ser-Pro, (c) Pro-Arg-Pro-Pro·!·Ala-Pro, (d) Pro-Pro·!·Thr-Pro, (e) Ala-Pro-Arg-Pro-Pro·!·Thr-Pro oder (f) Pro-Ala-Pro-Arg-Pro-Pro·!· Thr-Pro, wobei ·!· die Spaltstelle angibt.

Die vorliegende Erfindung beinhaltet insbesondere auch ein Protein bzw. Peptid mit der Aminosäuresequenz Met-Y-Pro·!·X-Pro-A, worin X vorzugsweise für Thr, Ala oder Ser steht, Y eine oder mehrere beliebige Aminosäuren bedeutet, und vorzugsweise, wenn X für Thr oder Ala steht, mit Pro endet oder, wenn X für Ser steht, mit der Sequenz Pro-Ala oder Pro endet, und A eine beliebige Aminosäuresequenz bedeutet. Ein derartiges Protein bzw. Peptid wird nach dem erfindungsgemäßen Verfahren durch Transformation einer prokaryontischen Zelle mit einem rekombinanten Vektor exprimiert, der mindestens eine Kopie eines Gens enthält, das für ein derartiges Protein bzw. Peptid kodiert.
Die Sequenz A kann für eine beliebige Aminosäuresequenz stehen. Günstigerweise befindet sich jedoch innerhalb dieser Aminosäuresequenz keine weitere Spaltstelle für die IgA-Protease.

Weiterhin ein Gegenstand der vorliegenden Erfindung ist eine rekombinante DNA, die für ein erfindungsgemäßes Protein bzw. Peptid kodiert und worin in mindestens einer Übergangsregion des fusionierten Proteins eine oder mehrere IgA-Protease-Erkennungsstellen bzw. Spaltsequenzen eingebaut sind.

Eine erfindungsgemäße rekombinante DNA ist auf eine dem Fachmann auf dem Gebiet der Molekularbiologie bekannte Weise erhältlich. Üblicherweise wird hierzu ein Vektor, der eine für die Aminosäuresequenz A kodierende DNA-Sequenz enthält, mit Restriktionsendonuklease(n) im Bereich des 5'-Ende dieses Gens gespalten und mit Oligonukleotiden, welche die gewünschte Sequenz enthalten, religiert. Das Oligonukleotid muß dabei eine Sequenz enthalten, die für eine Spaltstelle der IgA-Protease oder einem Teil davon kodiert.

Weiterhin ein Gegenstand der Erfindung ist auch ein rekombinanter Vektor, der mindestens eine Kopie einer erfindungsgemäßen rekombinanten DNA enthält. Die zur Proteinexpression in prokaryontischen Organismen geeigneten Basisvektoren sind dem Fachmann bekannt. Dieser Vektor ist günstigerweise ein solcher, der eine hohe Expression der erfindungsgemäßen rekombinanten DNA erlaubt. Vorzugsweise befindet sich die rekombinante DNA auf dem Vektur unter Kontrolle eines induzierbaren Expressionssignals (z.B. X, tac, lac oder trp Promotor).

Der erfindungsgemäße Vektor kann sowohl extrachromosomal (z.B. Plasmid) als auch im Genom des Wirtsorganismus integriert (z.B. Bakteriophage Lambda) vorliegen. Vorzugsweise ist der erfindungsgemäße Vektor ein Plasmid. Vektoren, die sich jeweils für die Genexpression in einem bestimmten Wirtsorganismus eignen, sind dem Fachmann auf dem Gebiet der Molekularbiologie bkeannt. Es kann sich um einen eukaryontischen, vorzugsweise aber um einen prokaryontischen Vektor handeln. Beispiele für zur Expression der erfindungsgemäßen DNA in Prokaryonten geeignete Vektoren sind etwa kommerziell erhältliche pUC- und pUR-Vektoren.

Ein weiterer Gegenstand der Erfindung ist eine Zelle, vorzugsweise eine prokaryontische Zelle, besonders bevorzugt eine E. coli Zelle, welche mit der erfindungsgemäßen rekombinanten DNA oder/und einem erfindungsgemäßen rekombinanten Vektor transformiert ist.

Beispiele für Proteine, welche die N-terminale Sequenz X-Pro besitzen, wobei X Thr, Ala oder Ser bedeutet, und die durch das erfindungsgemäße Verfahren in einem Schritt gewonnen werden können, sind etwa menschliches Erythropoietin, die β-Kette des menschlichen T-Zell-Rezeptors und insbesondere der menschliche Granulozyten-stimulierende Faktor (G-CSF).

G-CSF wird als Lymphokin von aktivierten Monozyten, Makrophagen sowie von einer Reihe anderer Zellinien synthetisiert. Lymphokine sind an der Reifung von Zellen des Immun- bzw. Blutzellensystems beteiligt. Sie stimulieren die Reifung von Knochenmark-Stammzellen zu ausdiffenzierten Zellen. So induziert G-CSF z.B. die Bildung von Neutrophilen und Granulozyten.

Da G-CSF in der Lage ist, die Population von neutrophilen Zellen innerhalb kurzer Frist erheblich zu steigern, ergeben sich für G-CSF beträchtliche therapeutische Anwendungsfelder. So könnte G-CSF z.B. nach Chemotherapie bei Krebs, bei der die Zellen des Immunsystems zerstört werden, eingesetzt werden. Weiterhin könnte man G-CSF bei Knochenmark-Transplantationen, bei schweren Brandwunden, bei durch Immunschwäche bedingten opportunistischen Infektionen und bei Leukämie verwenden.

G-CSF ist ein sekretorisches Proteinmolekül. Das primäre Translationsprodukt enthält daher eine N-terminale Signalsequenz, die bei der Sekretion abgespalten wird, so daß die Sequenz von reifem G-CSF mit den Aminosäuren Thr(+1)-Pro(+2) (Aminosäurepositionen +1 und +2) beginnt. Bei der Produktion von G-CSF in Prokaryonten wird dieses Signalpeptid nur schlecht oder gar nicht abgespalten, so daß zur Bereitstellung von G-CSF ohne Signalsequenz aus Prokaryonten ein AUG(Met) als Initiationscodon vor den Beginn der für reifen G-CSF kodierenden DNA-Sequenz, die auf Proteinebene mit Thr(+1)-Pro(+2) beginnt, kloniert werden muß. Als Folge davon wird in Prokaryonten, wie E.coli, ein G-CSF exprimiert, das ein Methionin an Aminosäureposition -1 enthält.

Durch das erfindungsgemäße Verfahren kann ein in Aminosäureposition -1 methioninfreier G-CSF aus Prokaryonten, der mit den Aminosäuren Thr(+1)-Pro(+2) beginnt, auf einfache Weise hergestellt werden.

Dies geschieht, indem man ein Derivat eines G-CSF aus Prokaryonten gewinnt, welches in Position +1 und +2 der Aminosäure-Sequenz die Aminosäuren Thr(+1)-Pro(+2) und davor ab Position -1 der Aminosäuresequenz eine Aminosäuresequenz enthält, welche von IgA-Protease erkannt und von der Aminosäuresequenz des G-CSF, welche mit Thr(+1)-Pro(2+) beginnt, abgespalten werden kann.

In einer bevorzugten Ausführungsform enthält das Derivat in Position -1 und -2 je ein Pro, in Position -3 bis Position -1 die Aminosäuresequenz Arg-Pro-Pro, in Position -4 bis -1 der Aminosäuresequenz Pro-Arg-Pro-Pro oder in Position -5 bis Position -1 die Aminosäuresequenz Ala-Pro-Arg-Pro-Pro.

In einer besonders bevorzugten Ausführungsform enthält das Derivat von Position -6 bis Position -1 die Aminosäuresequenz

-6  -5  -4  -3  -2  -1
Pro-Ala-Pro-Arg-Pro-Pro.

Unter G-CSF im Sinne der Erfindung wird natürlich vorkommendes G-CSF, dessen Sequenz beispielsweise in Science 232 (1986) 61 offenbart ist, sowie davon abgeleitete Derivate mit Granulozyten-stimulierender Wirksamkeit, deren Aminosäuresequenzen mit X(+1)-Pro(+2) beginnen, verstanden. X steht für Thr, Ser oder Ala, besonders bevorzugt für Thr.

Das erfindungsgemäße G-CSF-Derivat kann nach Expression in Prokaryonten durch Behandlung mit IgA-Protease zwischen Position +1 und -1 (zwischen Thr(+1) und Pro(-1)) gespalten werden. Damit wird durch einen einzigen Hydrolyseschritt ein in Position -1 methioninfreier G-CSF erhalten, dessen Aminosäuresequenz N-terminal mit den Aminosäuren Thr(+1)-Pro(+2) des natürlich vorkommenden G-CSF beginnt.

Bei der Expression von G-CSF in Prokaryonten werden schwerlösliche Aggregate (refractile bodies) gebildet, die inaktiv sind. Bevor das Protein z.B. für therapeutische Zwecke verwendet werden kann, muß es in seine aktive Form übergeführt werden. Bei den dem Fachmann geläufigen Verfahren (vgl. z.B. EP-A 0 219 874, EP-A 0 114 506, WO 84/03711) wird zunächst eine Solubilisierung durch Zugabe von Denaturierungsmitteln durchgeführt, an die sich eine Renaturierung und gegebenenfalls weitere Reinigungsschritte anschließen. Die Behandlung des erfindungsgemäßen Proteins mit IgA-Protease kann vor Solubilisierung, nach Solubilisierung oder erst nach der Renaturierung erfolgen. Falls direkt nach Solubilisierung die Behandlung mit IgA-Protease durchgeführt werden soll, muß das Solubilisierungsmittel (z.B. Guanidin-Hydrochlorid oder Harnstoff) durch Dialyse vor Zugabe der IgA-Protease abgetrennt werden. Vorzugsweise wird die Behandlung mit IgA-Protease jedoch nach Renaturierung durchgeführt, da in diesem Fall die Ausbeuten an G-CSF besonders hoch sind.

Die Bedingungen für die Behandlung von G-CSF bzw. eines anderen zu spaltenden Proteins mit IgA-Proteasen sind an sich unkritisch. Es ist jedoch bevorzugt, dabei G-CSF (bzw. ein anderes Protein) zu IgA-Protease im Gewichtsverhältnis 1 : 1 bis 100 : 1 einzusetzen. Die Umsetzung erfolgt bevorzugt in gepufferter wässriger Lösung von pH 6,5 bis 8,5. Die Pufferkonzentration liegt vorzugsweise im Bereich zwischen 50 und 300 mmol/l, gegebenenfalls unter Zusatz von 20 - 100 mmol/l Natriumchlorid. Vorzugsweise erfolgt die Spaltung bei Raumtemperatur über 20 - 60 Minuten.

Nach Solubilisierung, Renaturierung und Spaltung mit IgA-Protease wird das so erhaltene Spaltprodukt vorzugsweise über Ionentauscher und Größenfraktionierung gereinigt.
Die Verunreinigung des auf diese Weise hergestellten und in Position -1 methioninfreien G-CSF durch andere Proteine liegt unter 0,1 %, vorzugsweise unter 10⁻³ %.

In position -1 methioninfreies G-CSF kann durch Spaltung mit IgA-Protease also praktisch quantitativ von dem Methionin enthaltenen Fusionsprotein getrennt bzw. gereinigt werden.

Durch das erfindungsgemäße Verfahren kann ein rekombinanter G-CSF aus Prokaryonten erhalten werden, der zu weniger als 0,1 %, vorzugsweise zu weniger als 10⁻³ % durch anderere Proteine verunreinigt und quantitativ frei von einem G-CSF aus Prokaryonten ist, welcher in Position -1 ein Methionin enthält.

Ein Gegenstand der Erfindung ist auch ein Arzneimittel auf Basis eines durch das erfindungsgemäße Verfahren gewonnenen G-CSF aus Prokaryonten als Wirkstoff, gegebenenfalls zusammen mit üblichen pharmazeutischen Träger-, Füll- und Hilfstoffen. Eine solches Arzneimittel ist insbesondere für therapeutische Behandlungen, bei denen die Bildung von Granulozyten, insbesondere von Neutrophilen angeregt werden soll, geeignet.

Die erfindungsgemäßen pharmazeutischen Präparate kommen vorzugsweise als Injektions- und Infusionslösungen zum Einsatz. Dies kann dadurch geschehen, daß eine bereits spritzfertige Lösung zur Verfügung gestellt wird, die die erfindungsgemäße Zusammensetzung besitzt. Es ist jedoch auch möglich, die pharmazeutischen Präparate in Form von Lyophilisaten zur Verfügung zu stellen. Diese werden dann mit an sich bekannten, zu Injektionszwecken geeigneten Mitteln oder Lösungen rekonstituiert. Als Injektionsmedium kommt vorzugsweise Wasser zur Anwendung, welches die bei Injektionslösungen üblichen Zusätze wie Stabilisierungsmittel, Lösungsvermittler, Puffer und isotonische Zusätze, beispielsweise eine physiologische NaCl-Konzentration, enthält. Derartige Zusätze sind beispielsweise Mannit, Tartrat- oder Citrat-Puffer, Ethanol, Komplexbildner wie z. B. Ethylendiamintetraessigsäure und deren nicht-toxischen Salze, sowie hochmolekulare Polymere, wie flüssiges Polyethylenoxid zur Viskositätsregulierung. Flüssige Trägerstoffe für Injektionslösungen müssen steril sein und werden vorzugsweise in Ampullen abgefüllt.

Schließlich beinhaltet die vorliegende Erfindung auch die Verwendung von in Position -1 methioninfreiem G-CSF aus Prokaryonten zur Herstellung von erfindungsgemäßen pharmazeutischen Präparaten.

Für den Fall, daß man bei Spaltung eines beliebigen fusionierten Proteins durch das erfindungsgemäße Verfahren als resultierendes Produkt ein Protein X-Pro-A erhält (wobei X eine beliebige Aminosäure udn A eine beliebige Sequenz von Aminosäuren bedeutet), welches an seinem Aminoterminus, jedoch ein unerwünschtes Dipeptid X-Pro trägt, dann kann dieses unerwünschte Dipeptid als Teil des erfindungsgemäßen Verfahrens durch die weitere Behandlung mit Dipeptidylaminopeptidasen (DPAP) abgetrennt werden. Dipeptidylaminopeptidasen wurden bisher bei einer Reihe von Mikroorganismen, Insekten, Amphibien und in verschiedenen menschli- chen Geweben gefunden. Sie dienen z.B. zur schrittweisen Pro-zessierung von Vorläuferproteinen und besitzen zum Teil ausgeprägte Spezifität für den aminoterminalen Abbau des Dipeptids X-Pro (X-Pro-DPAPase; G. Kreil, Trends in Biochemical Sciences 15, 23-26, 1990). Durch die erfindungsgemäße Kombination von Igase und X-Pro-DPAP können daher erwünschte Proteine mit beliebigen aminoterminalen Aminosäuren hergestellt werden.

Mit der oben beschriebenen Kombination von Igase und X-Pro-DPAP gelingt es nun, auch Proteine mit einer anderen N-terminalen Aminosäuresequenz aus Prokaryonten in Position -1 methioninfrei herzustellen. Dazu wird zunächst ein Fusionsprotein gewonnen, welches die Aminosäuresequenz Met-Y-Pro·!·X-Pro-A besitzt, wobei nun in diesem Falle die Aminosäuresequenz A ohne die beiden N-terminalen Aminosäuren X-Pro der erwünschte Anteil des zu exprimierenden Proteins ist.

Das Expressionsprodukt der prokaryontischen Zelle mit der Aminosäuresequenz Met-Y-Pro·!·X-Pro-A wird zunächst mit IgA-Protease gespalten, so daß ein erstes Spaltprodukt mit der Aminosäuresequenz X-Pro-A entsteht:.

Dieses Protein kann nun, wie oben beschrieben, mit einer Dipeptidylamino-Peptidase behandelt werden, welche spezifisch die Sequenz X-Pro erkennt und hinter Pro spaltet. Somit entsteht ein zweites Spaltprodukt mit der an sich beliebigen Aminosäuresequenz A. Das erfindungsgemäße Verfahren erweist sich somit als äußerst nützlich zur Herstellung verschiedenster Proteine ohne N-terminalen Methionin-Rest und ist nicht nur auf die Herstellung von Proteinen mit der N-terminalen Sequenz X-Pro beschränkt, wobei X vorzugsweise für Ser, Thr oder Ala steht. ,

Schließlich beinhaltet die Erfindung auch eine rekombinante DNA, die einen für eine (oben definierte) IgA-Protease-Erkennungsstelle kodierten Bereich enthält und zum Einbau in eine Übergangsstelle von fusionierten Proteinen geeignet ist. Vorzugsweise handelt es sich dabei um ein chemisch synthetisiertes DNA-Fragment, an dessen Enden sich günstigerweise eine oder mehrere geeignete Restriktionsschnittstellen befinden.

### Begriffsdefinitionen:

Das erfindungsgemäße Verfahren beinhaltet die biotechnologische Gewinnung erwünschter Proteine. Unter biotechnologisch versteht sich in diesem Zusammenhang, daß ein erwünschtes Protein oder ein Zwischenprodukt desselben unter Verwendung gentechnologischer Mittel und anderer biotechnologischer Verfahren (z.B. der Fermentation von Mikroorganismen) hervorgeht.

Ein erwünschtes Protein ist ein Zwischenprodukt oder ein Endprodukt, das z.B. im Bereich der Medizin, der Forschung, im Umweltschutz oder bei industriellen Prozessen bzw. Produkten Verwendung finden kann.

Das erfindungsgemäße Verfahren beinhaltet, daß ein erwünschtes Protein aus einem fusionierten Protein (auch als Proteinfusion bezeichnet) gebildet wird, wobei sich das fusionierte Protein bzw. die Proteinfusion aus mehreren kovalent miteinander verbundenen Fusionspartnern zusammensetzt. Dabei stellt wenigstens einer der Fusionspartner ein erwünschtes Protein dar. Die Reihenfolge der Fusionspartner und deren Widerholungsgrad in einem fusionierten Protein ist beliebig; vorzugsweise besteht sie jedoch aus einem aminoterminalen Trägerprotein und einem carboxyterminalen erwünschten Protein.

Ein Trägerprotein bzw. ein Trägeranteil dient dazu, dem erwünschten Protein in Form eines fusionierten Proteins besondere Eigenschaften zu verleihen. Derartige Eigenschaften können sich beispielsweise in einer erhöhten Stabilität der fusionierten Proteine äußern, die auf strukturellen Besonderheiten und damit einhergehend höherer Resistenz gegenüber zellulären Proteasen oder aber auf dem Transport der fusionierten Proteine in eine Umgebung mit geringer proteolytischer Aktivität beruhen. Darüber hinaus können im Trägerprotein Eigenschaften enthalten sein, die eine effiziente Reinigung der fusionierten Proteine erlauben. Dazu gehört z.B. die Bindung von bestimmten Liganden in Zusammenhang mit affinitätschromatographischen Methoden, die Ablagerung der fusionierten Proteine in mit geringen Aufwand abtrennbaren Präzipitationskörpern und der Transport der Proteine an leicht zugängliche Orte.

Die Bereiche innerhalb eines fusionierten Proteins, in denen die Komponenten (Trägerproteine und erwünschte Proteine) eines fusionierten Proteins miteinander in Verbindung treten, werden als Übergangsregionen bezeichnet.

Eine jede Übergangsregion kann durch eine oder mehrere Aminosäureabfolgen definiert sein. Die Aminosäureabfolgen (wie auch alle übrigen Folgen von Aminosäuren und Proteinen) werden von aminoterminal (links) in Richtung carboxyterminal (rechts) verstanden und dargestellt.

Im Rahmen des erfindungsgemäßen Verfahrens enthalten all diejenigen Aminosäureabfolgen in Übergangsregionen, die durch IgA-Proteasen spaltbar sein sollen, die erfindungsgemäße Spaltsequenz bzw. Erkennungssequenz.

Diejenige Stelle zwischen zwei Aminosäuren einer Aminosäureabfolge, an der die Spaltung von fusionierten Proteinen bzw. Proteinfusionen erfolgt, wird als Spaltstelle bezeichnet.

Das erfindungsgemäße Verfahren beinhaltet die enzymatische Spaltung von fusionierten Proteinen in den Übergangsregionen durch IgA-Protease. Unter IgA-Protease bzw. Igase werden im Rahmen des erfindungsgemäßen Verfahrens die IgA-Protease des Stammes Neisseria gonorrhoeae MS11 und alle anderen mit dieser Protease auf Nukleotidebene und in ihrem Bildungsprozeß verwandten Enzyme verstanden. Hierunter zählen insbesondere auch die IgA-Proteasen der Genera Neisseria und Haemophilus.

Der Mikroorganismus E.coli ED 8654 wurde unter der Nummer DSM 2102 bei der Deutschen Sammlung für Mikroorganismen, Griesebachstraße 8, 3400 Göttingen hinterlegt.

Die Erfindung wird durch die folgenden Beispiele in Verbindung mit der Zeichnung erläutert.

In der Zeichnung stellen dar:

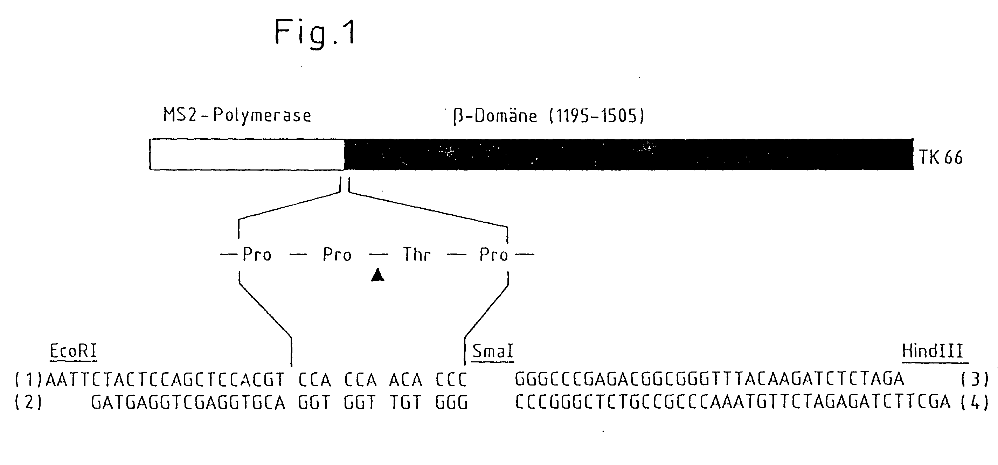

- Figur 1: zeigt schematisch die Proteinfusion zwischen dem Trägerprotein MS2-Polymerase (99 Aminosäuren) und der β-Domäne (Aminosäureposition 1195-1505) des IgA-Protease-Vorläufers aus N. gonorrhoeae MS11. Die Übergangsregion zwischen diesen beiden Anteilen besteht aus 12 Aminosäuren und enthält die Spaltsequenz -Pro-Pro·!·Thr-Pro- für Igase. Für die Konstruktion der Spaltstelle wurden die vier Oligonukleotide (1) bis (4) zwischen die Restriktions-schnittstellen Eco RI und HindIII eingebaut. Die Herstellung des Polypeptids und die Spaltung mit gereinigter Igase sind unter Beispiel 5 näher erläutert.

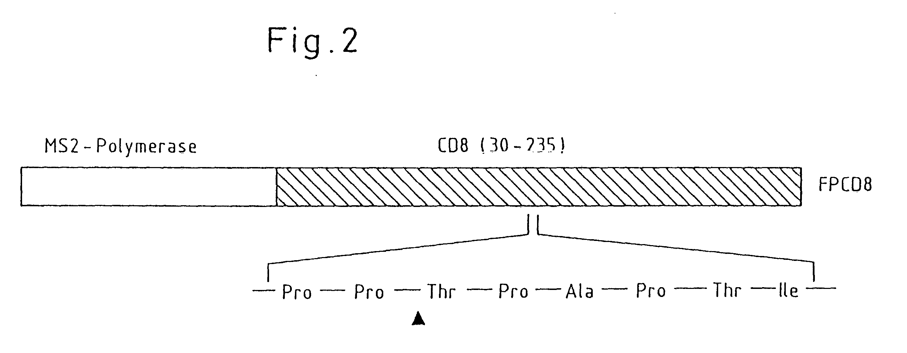

- Figur 2: zeigt eine Proteinfusion bestehend aus dem Träger-protein (99 Aminosäuren der MS2-Polymerase und 6 Plasmid-kodierte Aminosäuren) und 206 Aminosäuren vom CD8-Protein menschlicher T-Lymphozyten. In der Aminosäureabfolge des CD8-Proteins befindet sich eine natürliche Spaltsequenz, die durch Igase gespalten wird (siehe Beispiel 6).

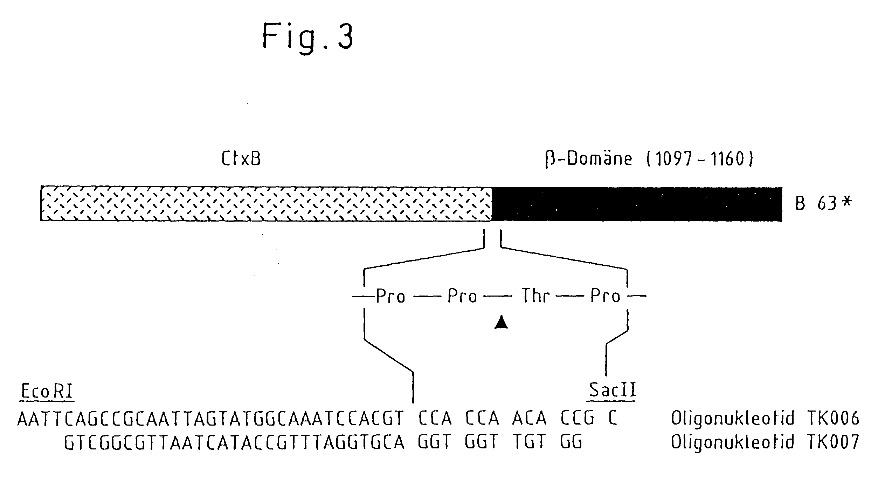

- Figur 3: zeigt die Proteinfusion B63*, die mittels eines Expressions-Sekretionssystems von E.coli-Zellen produziert wurde. Sie besteht am Aminoende aus der Choleratoxin B Untereinheit (103 Aminosäuren), gefolgt von einer Verbindungsregion (11 Aminosäuren) mit der Igase Spaltstelle und am Carboxylende einem Teil (Aminosäureposition 1097-1160) der β-Domäne des IgA-Protease-Vorläufers. Die Igase Schnittstelle wurde mittels der beiden Oligonukleotide Tk006 und Tk007 zwischen die beiden Proteindomänen eingebaut.

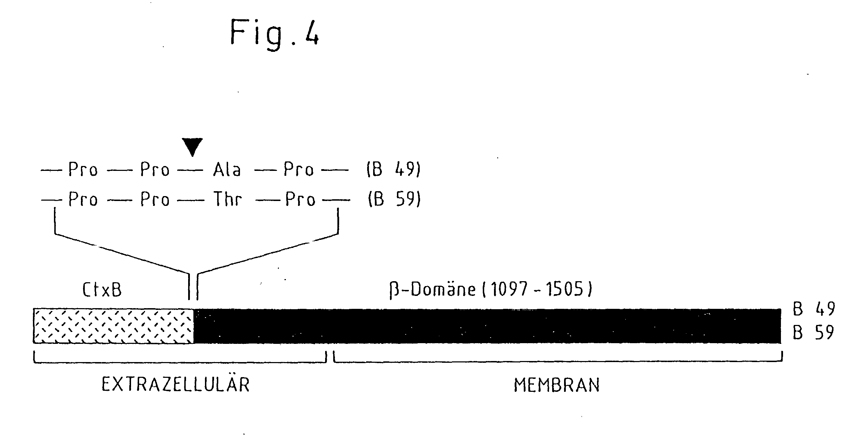

- Figur 4: zeigt schematisch die Proteinfusionen B49 und B59. Sie bestehen aus der Choleratoxin B Untereinheit und der β-Domäne des IgA-Protease-Vorläufers. Zwischen diesen Anteilen enthalten sie zwei unterschiedliche Übergangsregionen mit zwei verschiedenen Igase-Spaltsequenzen (-Pro-Pro-Ala-Pro- und - Pro-Pro-Thr-Pro-). Die Spaltsequenz wurde mit synthetischen Oligonukleotiden konstruiert (siehe Figur 3).

### Beispiel 1

### Konstruktion eines Plasmids zur Expression eines Methionin freien G-CSF.

Die Konstruktion wird unter Verwendung des Expressionsvektors pPZ07-mgllac (WO, 88/09373) durchgeführt. Dazu wird der Expressionsvektor pPZ07-mgllac mit Nco I gespalten und die überstehenden Enden mit Mung bean Nuklease entfernt. Anschließend wird der Vektor mit Bam Hl nachgespalten. Die IgA Erkennungssequenz wird auf DNA-Ebene über folgende Oligonukleotide bereitgestellt.

Oligonukleotid A:
5′ AAT TCG GAG GAA AAA TTA ATG ACA CCA CTG CGA CCT CCT ACA
CCA CTG GGC CCT G 3′

Oligonukleotid B
5′ GAT CC AGG GCC CAG TGG TGT AGG AGG TCG CAG TGG TGT CAT TAA
TTT TTC CTC CGA ATT 3′

Die beiden Oligonukleotide werden in äquimolaren Mengen zusammengegeben und in ca. einem 100-fachen Überschuß in den wie oben beschriebenen gespaltenen Vektor pPZ07-mgllac eingeführt. Nach Religierung werden auf übliche Weise kompetent gemachte Zellen von E.coli K12 transformiert. Nach bekannten Methoden wird die DNA aus den Zellen isoliert und mit Apa I und Bam HI gespalten. Ein ca. 520 bp langes G-CSF- Fragment wird unter Verwendung der Restriktionsendonukleasen Apa I und BamHI aus der G-CSF Sequenz (Science 232 (1986), 61-65) isoliert. Dieses Fragment wird in den ebenfalls mit ApaI und BamHI gespaltenen Vektor einligiert.

### Beispiel 2

Zusätzlich zu der IgA1-Erkennungssequenz kann das Fusionsprotein auch noch Peptide als Reinigungshilfe enthalten. Diese können aus der DNA die Stzeptavidin kodiert bestehen. Dazu wird ein Streptavidin Gen (WO 89/03422) in das richtige Translationsraster vor die IgA-Frotease Erkennungssequenz kloniert.

### Beispiel 3

Mit dem in Beispiel 1 beschriebenen Plasmid werden E.coli K12-Zellen (ED 8654, DSM 2102) transformiert, auf den Antibiotikamarker (Ampicillin) selektioniert und das Plasmid durch Restriktionsanalyse charakterisiert. Ein solcher Klon wird zur Anzucht und Expression von G-CSF verwendet. Die Zellen werden einem Vollmedium angezogen. Dieses Medium enthält pro Liter 16 g Bactotrypton (Difco), 10 g Hefe Extrakt (Difco) und 5 g Natriumchlorid. Die Zellen werden bis zu einer OD 546 von 2,0 wachsen gelassen und dann mit 10⁻³ mol/l IPTG induziert. Nach weiteren 4 h werden die Zellen durch Zentrifugation geerntet, mit Lysozym/EDTA aufgeschlossen und das G-CSF als inclusion bodies (::B's, vgl. EP-A 0 219 874) isoliert.

Die Denaturierung bzw. Renaturierung der isolierten unlöslichen G-CSF-Fusionspartikel wird, wie in EP-A 0 219 874 beschrieben, durchgeführt. Die Denaturierung erfolgt durch Dialyse gegen 6 mol/l Guanidin-Hydrochlorid. Es kann hier bereits ein Aliquot entnommen werden und nach Dialyse gegen 5 mmol/l Kaliumphosphatpuffer pH 7 für die Spaltung mit IgA-Protease (Bsp. 4) eingesetzt werden.

Alternativ erfolgt nach der Denaturierung in Guanidin-Hydrochlorid eine Dialyse gegen einen 5 mmol/l Kaliumphosphatpuffer pH 7 der 1 mmol/l GSH und 3 mmol/l GSSG enthält. Nach Renaturierung erfolgt hier ebenfalls eine Dialyse gegen 5 mmol/l Kaliumphosphatpuffer pH 7.

### Beispiel 4

### Spaltung des Fusionsprotein mit IgA1-Protease zur Herstellung eines nativen G-CSF ohne Methionin in Position-1

IgAl-Protease wird wie in EMBO Jour. 3 (1984), 1595-1601 beschrieben isoliert. Zu 10 µg nach Beispiel 3 renaturiertem bzw. denaturiertem G-CSF werden 2-5 µg IgA-Protease gegeben und 30 min bei Raumtemperatur inkubiert. Über verschiedene Ionenaustauschersäulen wie Mono-Q oder Mono-S kann das methioninfreie G-CSF isoliert werden. Proteinsequenzierung des aminoterminalen Endes ergibt, daß das gereinigte G-CSF mit der korrekten Aminosäurefolge Thr(+1)-Pro(+2) beginnt.

### Beispiel 5

### Herstellung einer Proteinfusion und Spaltung unlöslicher Protein-Aggregate aus "Inclusion Bodies" an einer Igase spezifischen Schnittstelle.

Der prokaryontische Expressionsvektor pEX31C (K. Strebel, Journal of Virology 57, 983-991, 1986) wurde in solcher Weise modifiziert, daß eine mit diesem System in E.coli-Zellen überproduzierte Proteinfusion durch Igase in das Trägerprotein und das erwünschte Protein aufgespalten werden konnte. Hierfür wurde aus synthetisch hergestellten Oligonukleotiden ein doppelsträngiges DNA-Fragment zusammengesetzt, welches für die Aminosäuresequenz Thr-Pro-Ala-Pro-Arg-Pro-Pro·!·Thr-Pro kodiert. Dieses DNA-Fragment wurde mit gentechnologischen Methoden in die EcoRI-Schnittstelle des Expressionsvektors pEX31C eingesetzt. Darüber hinaus wurden direkt angrenzend in die HindIII-Schnittstelle zwei weitere synthetische DNA-Fragmente eingefügt, welche eine Reihe geeigneter Schnittstellen für Restriktions-Endonukleasen und Terminations-Signale für die an der Genexpression beteiligten bakteriellen Enzyme enthielten. In das so entstandene Expessionsplasmid pEV37 wurde unter Benutzung der Schnittstellen für SmaI und HindIII ein DNA-Fragment eingesetzt, das für die β-Domäne des IgA-Protease-Vorläuferproteins aus Neisseria gonorrhoeae MS11 kodiert. Dadurch entstand ein hybrides Gen, bei dessen Ausprägung in E.coli ein Fusionsprotein gebildet wurde. Dieses enthielt am Aminoende als Trägerprotein 99 Aminosäuren der MS2-Polymerase, gefolgt von einer zentralen Übergangsregion von 12 Aminosäuren mit der Igase Spaltsequenz und der gewünschten β-Domäne am Carboxylende (siehe Figur 1). Mit gereinigter Igase ließ sich die β-Domäne vom Carboxylende der Proteinfusion an der Spaltstelle Pro·!·Thr innerhalb der Übergangsregion abspalten.

Das Plasmid mit dem hybriden Gen wurde durch Transformation in E.coli Zellen eingebracht, die für die regulierbare Überprodukticn der Proteinfusion den Regulationsfaktor CI⁸⁵⁷ aus dem Bakteriophagen Lambda enthielten (E. Remaut, Gene 22, 103-113, 1983). Durch Temperaturerhöhung von 28°C auf 42°C wurde der CI ⁸⁵⁷-Repressor inaktiviert und infolgedessen die Proteinproduktion in den rekombinanten E.coli-Zellen aktiviert. Hierzu wurden 50 ml einer 12 h bei 28°C gewachsenen E.coli-Kultur in 200 ml, zuvor auf 45°C vorgewärmtes Medium überführt und 2 Stunden bei 42°C weiter kultiviert. In diesem Schritt wurde die Proteinfusion im Zytoplasma der Bakterien in großen Mengen in Form von "Inclusion Bodies" angereichert. Die Bakterien wurden danach durch Zentrifugation geerntet, in 20 ml Lysepuffer (10 % Saccharose, 50 mM Tris/HCl pH 8,0, 1 mM EDTA) suspendiert und nach Zugabe von 400 µl Lysozymlösung (5 mg/ml) 30 min bei 22°C inkubiert. Das Detergenz Triton X-100 wurde bis zu einer Endkonzentration von 0,1 % zugegeben und die Lösung erneut 30 min inkubiert. Die bei der Lyse der Zellen freigesetzte DNA wurde durch Ultraschall-Behandlung zerkleinert, die unlöslichen Bestandteile, u.a. die in Präzipitationskörpern vorliegende Proteinfusion, wurden abzentrifugiert und anschließend in 5 ml H1NTE-Puffer (1 M Harnstoff, 50 mM NaCl, 50 mM Tris/HCl pH 8,0, 1 mM EDTA) gewaschen. Nach erneutem Abzentrifugieren wurde das Sediment durch Beschallen in 5 ml PBS-Puffer (20 mM Kaliumphosphat, pH 7,5, 140 mM NaCl) suspendiert und gewaschen. Dieser Vorgang wurde mehrfach wiederholt, um Harnstoff-Rückstände vollständig zu entfernen. Schließlich wurde die unlösliche Fraktion, die das Fusionsprotein enthielt, durch Beschallen in 5 ml PBS-Puffer suspendiert.

Die Qualität und die Menge der suspendierten Proteinfusion wurde mittels SDS-Polyacrylamid-Gelelektrophorese (12,5 %) und anschließender Färbung mit Coomassie-Blau bestimmt. Zur Spaltung wurde die Proteinsuspension in einem Enzym/Substrat-Verhältnis von 1/100 (W/W) 3 h bei 37°C inkubiert. Die erfolgte Spaltung der ungereinigten und unlöslichen Proteinfusion wurde durch analytische SDS-Polyacrylamid-Gelelektrophophorese untersucht, wobei sich ergab, daß ein Polypeptid in der für das β-Protein erwarteten Größe entstanden war. Dieses Protein wurde auf eine Nitrozellulosemembran übertragen und einer automatisierten Sequenzanalyse unterworfen. Die Abfolge der endständigen Aminosäuren bestätigte seine Entstehung aus der Proteinfusion durch korrekte Spaltung an der vorhandenen Igase-Spaltstelle.

Bei der Spaltung wurden jedoch nur bis zu etwa 50 % der Gesamtmenge des eingesetzten Substrats umgesetzt. Durch Zugabe größerer Mengen Igase und durch längere Reaktionszeiten konnte keine Steigerung erzielt werden. Dies deutet darauf hin, daß im ungeschnittenen Anteil des Fusionsproteins die Schnittstelle für Igase nicht zugänglich ist. Hybridprotein und Spaltprodukte lagen auch nach der Inkubation mit Igase weiterhin in Form unlöslicher Aggregate vor und ließen sich demzufolge aus der Suspension durch Zentrifugation sedimentieren.

Spaltungs-Ausbeuten bis zu 90 % wurden erzielt, wenn anstatt der unsauberen "Inclusion Body"-Fraktion eine Proteinfusion eingesetzt wurde, die zuvor einem zusätzlichen Aufreinigungsschritt unterworfen worden war. Dazu wurde das unlösliche Sediment nach dem Waschen in HINTE-Puffer (s.o.) in 5 ml H7NTE-Puffer (7 M Harnstoff, 50 mM NaCl, 50 mM Tris/HCl pH 8,0, 1 mM EDTA) aufgenommen. Unlösliche Anteile wurden durch Zentrifugation abgetrennt und die lösliche Fraktion gegen 5 l PBS-Puffer bei 4°C dialysiert. Bei der Entfernung des Harnstoffes durch Dialyse präzipitierte das Fusionsprotein aus der Lösung in Form von unlöslichen Aggregaten. Die ausgefallenen Aggregate wurden durch Ultraschall-Behandlung in eine feine Suspension überführt und wie oben beschrieben mit Igase gespalten und analysiert.

### Beispiel 6

### Spezifische Spaltung einer renaturierten, löslichen Proteinfusion mit Igase

Unter Verwendung des pEX-Expressionssystems wurde ein Hybridprotein hergestellt (siehe Beispiel 1), welches aus der MS2-Polymerase und einem Teil des CD8-Proteins menschlicher cytotoxischer T-Lymphozyten besteht (siehe Figur 2). Nach der Vorreinigung und dem Lösen des Proteins aus "Inclusion Bodies" in H7NTE-Puffer wurde als weiterer Reinigungsschritt ein präparatives 12,5 % SDS-Polyacrylamidgel eingesetzt. Die Proteinfusion wurde als einzelne Bande nach dem Färben mit Coomassie-Blau aus dem Gel ausgeschnitten und anschließend nach der Methode von Hunkapiller (Methods in Enzymology 91, 227-235, 1983) vom Gelmaterial getrennt. Die Elektroelution erfolgte dabei in TAE-Puffer (40 mM Tris/Acetat, pH 7,9) dem 0,1 % SDS (Natriumlaurylsulfat) zugesetzt war. Das SDS wurde später durch Dialyse gegen 5 l TAE-Puffer bei 22°C entfernt. In einer weiteren Dialyse wurde das Protein in Aufbewahrungspuffer (20 mM Kaliumphosphat, pH 7,5, 140 mM NaCl, 50 % Glycerin) überführt. Das somit erhaltene lösliche Fusionsprotein wurde mit gereinigter Igase inkubiert (siehe Beispiel 5) und dabei vollständig an einer im CD8-Molekül enthaltenen Spaltstelle (-Pro-Pro·!·Thr-Pro-Ala-, siehe Figur 2) in zwei Polypeptid-Fragmente gespalten. Die Spezifität der Spaltung wurde durch Analyse der Aminosäureabfolge am Aminoende des kleineren Spaltproduktes überprüft. Dabei ergab sich wie erwartet die Sequenz Thr-Pro-Ala-Pro-Thr-Ile.

### Beispiel 7

### Spezifische Spaltung einer löslichen, aus Kulturüberständen gewonnenen Proteinfusion mit Igase.

Eine Proteinfusion bestehend aus der Choleratoxin B Untereinheit und einem Teil der b-Domäne (Pos. 1097-1160; J. Pohlner, Nature 325, 458-462, 1987) des IgA-Protease-Vorläufers aus N.gonorrhoeae MS11 wurde in löslicher Form aus Kulturüberständen rekombinanter E.coli-Zellen gewonnen. Um die beiden Proteinanteile voneinander trennen zu können, war in die Übergangsregion zwischen der Choleratoxin B-Untereinheit und der β-Domäne mit gentechnologischen Methoden eine künstliche Spaltsequenz für Igase (Pro-Pro · ! · Thr-Pro-) eingesetzt worden. Hierfür waren die Oligonukleotide Tk006 und Tk007 zwischen die Restriktionsschnittstellen EcoRI und SacII in der Übergangsregion eingesetzt worden (siehe Figur 3). Das Fusionsprotein wurde aus 2 l Überstand einer 12 h bei 37°C gewachsenen Bakterienkultur durch Fällung mit Ammoniumsulfat angereichert und anschließend gegen 5 1 PBS-Puffer dialysiert. Zur Spaltung wurde es in einer Konzentration von 50 µg/ml in PBS-Puffer in einem Enzym/Substrat-Verhältnis von 1/50 (W/W) mit gereinigter Igase für 2 h bei 37°C inkubiert. Eine Immunblot-Analyse zeigte, daß das bei der vollständigen Spaltung auftretende größere Spaltfragment in seinem Molekulargewicht und in seiner Reaktion mit Antiserum der natürlichen Choleratoxin B-Untereinheit entsprach.

### Beispiel 8

### Spezifische Spaltung von Proteinfusionen auf der Oberfläche gramnegativer Bakterien mittels Igase.

Ein Expressions-Sekretionssystem wurde benutzt, um die Proteinfusionen TKB49 und TKB 59 bestehend aus der Choleratoxin B Untereinheit und der Iga-Protease β-Domäne auf der Oberfläche von rekombinanten Salmonellen nach außen zu exponieren. Das für TkB49 kodierende hybride Gen enthielt in der Übergangsregion zwischen der Toxin- und der β-Domäne die Original-Spaltsequenz (c) (-Pro-Pro·!·Ala-Pro-) für die Igase. Dagegen wurde in das Gen für TkB59 ein künstliches DNA-Fragment, bestehend aus den Oligonukleotiden Tk006 und Tk007 zwischen die Restriktionsschnittstellen EcoRI und SacII eingesetzt, das für die Spaltsequenz (-Pro-Pro·!·Thr-Pro-) kodierte (siehe Figur 3). Wurden intakte Bakterien, die solche an ihrer Oberfläche verankerten Proteinfusionen trugen, mit gereinigter Igase inkubiert, dann konnte die spezifische Spaltung an den Igase-Schnittstellen beobachtet werden. In Immunblot-Analysen wurde gezeigt, daß die bei der Spaltung auftretenden kleinen Spaltfragmente in ihrer Größe und Reaktion mit Antiserum der natürlichen Choleartoxin B Untereinheit entsprachen.

### Beispiel 9

### Aufreinigung aktiver Igase aus Kulturüberständen rekombinanter E.coli-Zellen

Rekombinante E.coli C600 Zellen, die das Plasmid pEX1070 (DE 36 22 221.6) mit einem modifizierten IgA-Proteasegen enthalten, sekretieren die aktive Igase in den Kulturüberstand. Durch Membranfiltration konnte das Enzym im Kulturüberstand angereichert und anschließend mit Ammoniumsulfat (0,42 g/ml) aus der Lösung ausgefällt werden. Nach Zentrifugation wurde das Sediment in Biorex-Puffer (50 mM Kaliumphosphat, pH 7,0, 8,6 % Glyzerin) gelöst (1 ml Puffer pro 1 l Kulturüberstand), durch Dialyse gegen 2 l Puffer äquilibriert und anschließend einer Kationenaustausch-Chromatographie (Biorex70) unterworfen. Die gebundene IgA-Protease wurde in einem Schritt mit Elutions-Puffer (500 mM Kaliumphosphat, pH 7,0, 8,6 % Glyzerin) von der Säule eluiert und fraktioniert. IgA-Protease enthaltende Fraktionen wurden anschließend durch SDS-Polyacrylamid-Gelelektrophorese (12,5 %) analysiert. An diesem Punkt der Aufreinigung ergab sich ein durchschnittlicher Reinheitsgrad von > 90 %. Für die Darstellung der Igase in reiner Form wurden eine Gelfiltration mit Sephacryl HR300 in Biorex-Puffer, gefolgt von einer weiteren Kationenaustausch-Chromatographie (s.o.), durchgeführt. Die Aktivität der Igase wurde durch Inkubation mit IgA 1-Antikörpern und Auftrennung der entstandenen Spaltprodukte im SDS-Polyacrylamidgel getestet.

### Beispiel 10

### Konstruktion eines Plasmides zur Expression eines methioninfreien Interleukin 3:

Die Konstruktion wird unter Verwendung des Expressionsvektors pPZ07-mglac (WO88/09373) durchgeführt. Dazu wird der Expressionsvektcr pPZ07-mgllac mit NCOK gespalten und die überstehenden Enden mit Mung bean Nuklease entfernt. Anschließend wird der Vektor mit Bam HI nachgespalten. Die optimierte aminoterminale Region des Fusionsproteins wird auf DNA-Ebene über folgende Oligonukleotide bereitgestellt:

Primer 1A:

5′ AATTCGGAGGAAAAATTAATGAAAGCCAAACGTTTTAAAAAACATGTCGACCATG
GAG 3′

Primer 1B:

5' GGATCCTCCATGGTCGACATGTTTTTTAAAACGTTTGGCTTTCATTAATTTTTCC -
TCCGAATT 3'

Beide Oligonukleotide werden in equimolaren Mengen zusammengegeben und in ca. 100-fachem Überschuss in den wie oben beschriebenen gespaltenen Vektor pPZ07-mgllac eingeführt. Nach Ligation werden auf übliche Weise kompetent gemachte Zellen von E.coli K12 transformiert. Nach bekannten Methoden wird die DNA aus den Zellen isoliert, mit SalI/Bam HI gespalten und mit einem DNA Fragment, das die kodierende Region von Interleukin 3 ohne Signalsequenz enthält (nachfolgend beschrieben), ligiert.

Die Bereitstellung der kodierenden Region von Interleukin 3 mit der Erkennungsregion für IgA Protease auf DNA-Ebene erfolgt über die bekannte Technik der PCR, indem eine PCR-Reaktion mit der c-DNA von Interleukin 3 als Template und mit nachstehend beschriebenen Primern durchgeführt wird:

Primer 2A:

5′ AAGCTTGTCGACCCACGTCCACCAGCTCCCATGACCCAGACAACGCCC 3′

Primer 2B:

5′ TTCGTTGGATCCCTAAAAGATCGCGAGGCTCAAAGT 3′

Das resultierende PCR Fragment wird mit den Enzymen Sal I und Bam HI nachgeschnitten und kann so direkt in die oben beschriebene Vektor-DNA eingefügt werden und kovalent mit Hilfe von Ligase verbunden werden.
Nach Transformation der DNA in einen geeigneten Wirt, z.B. E.coli K12 C600, kann Il 3 in E.coli in Form von Rb's synthetisiert und anschließend isoliert werden. Wie für G-CSF beschrieben wird eine De-und Renaturierung des Proteins durchgeführt und das renaturierte Protein mit IgA-Protease gespalten. Das so hergestellte met-freie Il 3 kann nach weiteren Reinigungsschritten zur Therapie eingesetzt werden.

### Beispiel 11:

### Konstruktion eines Plasmids zur Expression eines Methionin-freien Interleukin 2

Die Konstruktion kann unter Verwendung des Expressionsvektors pPZ07-mgllac (W088/09373) durchgeführt werden der nach Insertion der Primer 1A und 1B wie in Beispiel 10 beschrieben mit SalI/Bam HI gespalten wurde. Die Bereitstellung der kodierenden Region von Interleukin 2 mit der IgA-Protease Erkennungsregion auf DNA-Ebene erfolgt über die Methode der PCR, indem eine PCR-Reaktion mit der c-DNA von Interleukin 2 als Template und den Primern 3A und 3B, die ebenfalls die Erkennungsregion für die IgA-Protease kodieren, durchgeführt wird.

Primer 3A:

5' AAGCTTGTCGACCCACGTCCACCAGCACCTACTTCAAGTTCTACAAAG 3'

Primer 3B:

5′ TTCGTTGGATCCTCAAGTTAGTGTTGAGATGATGCTTT 3′

Das so erhaltene PCR-Fragment wird mit den Enzymen SalI und Bam HI nachgeschnitten und kann so direkt in die oben beschriebene Vektor DNA eingefügt werden. Das weitere Vorgehen erfolgt wie bei Il 3 beschrieben.In dem vorhergehenden Beispiel wurde beschrieben wie durch geeignete Verwendung der IgA-Protease Erkennungsschnittstelle und dem weiteren Verfahren Methionin-freie therapeutische Proteine hergestellt werden können, die mit der Aminosäuresequenz Ala Pro beginnen. Weitere Proteine, die analog den vorher beschriebenen Beispielen Methion-frei hergestellt werden können und zwar unter Verwendung von Oligonukleotiden die einmal die Erkennungsregion für die IgA-Protease sowie eine Region enthalten die analog dem 5' bzw. dem 3' Ende der publizierten Sequenz entspricht und damit über PCR-Amplifikation bereitgestellt werden können. Im folgenden sind weitere therapeutisch relevante Proteine aufgeführt, die in der reifen natürlicherweise vorkommenden Form mit Ala Pro beginnen und daher mit dem erfindungsgemäßen Verfahren in analoger Weise hergestellt werden können:
Cathepsin L (EC 3.4.22.15), Mason, R.W. et al. Biochem. J. 240, 373-377, 1986.
Erythropoietin, Lai, P.H. et al. J. Biol. Chem. 261, 3116-3121, 1986.
Interleukin-1 beta, Zsebo, K.M. et al. Blood 71, 962-968, 1988.
Osteonectin, Fisher, L.W. et al. J. Biol. Chem. 262, 9702-9708, 1987.
Type IV collagenase, Collier I.E. et al, J. Biol. Chem. 263, 6579-6587, 1988.

Weiterhin können auf diese Weise Proteine hergestellt werden, die in der reifen Form mit der Aminosäuresequenz Ser, Pro beginnen. Als Beispiel sind hier zu nennen:
Alpha-1 antitrypsin, Hill, R.E. et al., Nature 311 , 175-177, 1984.
Atrial natriuretic factor, Kambayashi, Y. et al., FEBS Lett. 259, 341-345, 1990.

Weitere Beispiele für Proteine die in ihrer reifen Form mit Thr Pro beginnen und die therapeutische relevant sein können sind zum Beispiel:
Complement factor B, Campell, R. D. et al., Proc. Nat. Acad. Sci. 80, 4464-4468, 1983.
Apolipoprotein A, Eaton, D.L. et al., Proc. Nat. Acad. Sci. 84, 3224-3228, 1987.

Nachstehend ist die Hinterlegungsangabe für den erwähnten Mikroorganismus aufgeführt.

## Claims (Claims for the following Contracting State(s): DE, FR, GB, IT, AT, BE, LU, NL, SE, DK, CH, LI)

1. Recombinant G-CSF or recombinant G-CSF derivative from prokaryotes,
**characterized in that**
the non-glycosylated, mature G-CSF or the G-CSF derivative which has the N-terminal amino acid sequence Thr-Pro is quantitatively free from a G-CSF from prokaryotes with an N-terminal methionine residue and is contaminated by less than 0.1 % by other proteins.

2. Recombinant G-CSF or G-CSF derivative as claimed in claim 1,
**characterized in that**
the G-CSF or the G-CSF derivative is contaminated by less than 10⁻³% by other proteins.

3. Pharmaceutical preparation containing a G-CSF or a G-CSF derivative as claimed in claim 1 or 2 as the active substance and optionally common pharmaceutical additives, auxiliary substances or/and carriers.

4. Use of a G-CSF or a G-CSF derivative as claimed in claim 1 or 2 to produce pharmaceutical preparations to which common pharmaceutical additives, auxiliary substances, fillers or/and carrier substances are optionally added.

## Claims (Claims for the following Contracting State(s): ES, GR)

1. Process for preparing a recombinant G-CSF or recombinant G-CSF derivative from prokaryotes,
**characterized in that**
the non-glycosylated, mature G-CSF or the G-CSF derivative which has the N-terminal amino acid sequence Thr-Pro is quantitatively free from a G-CSF from prokaryotes with an N-terminal methionine residue and is contaminated by less than 0.1% by other proteins.

2. Process for preparing a recombinant G-CSF or G-CSF derivative as claimed in claim 1,
**characterized in that**
the G-CSF or the G-CSF derivative is contaminated by less than 10⁻³% by other proteins.

3. Process for preparing a drug or pharmaceutical preparations containing a G-CSF or a G-CSF derivative as claimed in claim 1 or 2 as the active substance and optionally common pharmaceutical additives, auxiliary substances or/and carriers.

4. Use of a G-CSF or a G-CSF derivative as claimed in claim 1 or 2 to produce a drug or pharmaceutical preparations to which common pharmaceutical additives, auxiliary substances, fillers or/and carrier substances are optionally added.

## Patentansprüche (Patentansprüche für folgende(n) Vertragsstaat(en): DE, FR, GB, IT, AT, BE, LU, NL, SE, DK, CH, LI)

1. Rekombinanter G-CSF oder rekombinantes G-CSF-Derivat aus Prokaryonten, **dadurch gekennzeichnet, dass** der nicht-glykosylierte, reife G-CSF oder das G-CSF-Derivat mit der N-terminalen Aminosäuresequenz Thr-Pro quantitativ frei von einem G-CSF aus Prokaryonten mit N-terminalem Methioninrest ist und zu weniger als 0,1 % durch andere Proteine verunreinigt ist.

2. Rekombinanter G-CSF oder G-CSF-Derivat nach Anspruch 1, **dadurch gekennzeichnet, daß** der G-CSF oder das G-CSF-Derivat zu weniger als 10⁻³% durch andere Proteine verunreinigt ist.

3. Arzneimittel, enthaltend einen G-CSF oder ein G-CSF-Derivat nach Anspruch 1 oder 2 als Wirkstoff und gegebenenfalls übliche pharmazeutische Zusatz-, Hilfs- oder/und Trägermittel.

4. Verwendung eines G-CSF oder eines G-CSF-Derivats nach Anspruch 1 oder 2 zur Herstellung von pharmazeutischen Präparaten gegebenenfalls unter Zusatz von üblichen pharmazeutischen Zusatz,- Hilfs-, Füll- oder/und Trägerstoffen.

## Patentansprüche (Patentansprüche für folgende(n) Vertragsstaat(en): ES, GR)

1. Verfahren zur Herstellung eines rekombinanten G-CSF oder rekombinanten G-CSF-Derivats aus Prokaryonten,
**dadurch gekennzeichnet,**
**dass** der nicht-glykosylierte, reife G-CSF oder das G-CSF-Derivat mit der N-terminalen Aminosäuresequenz Thr-Pro quantitativ frei von einem G-CSF aus Prokaryonten mit N-terminalem Methioninrest ist und zu weniger als 0,1 % durch andere Proteine verunreinigt ist.

2. Verfahren zur Herstellung eines rekombinanten G-CSF oder G-CSF-Derivats nach Anspruch 1,
**dadurch gekennzeichnet,**
**dass** der G-CSF oder das G-CSF-Derivat zu weniger als 10⁻³ % durch andere Proteine verunreinigt ist.

3. Verfahren zur Herstellung eines Arzneimittels oder von pharmazeutischen Präparaten, enthaltend einen G-CSF oder ein G-CSF-Derivat nach Anspruch 1 oder 2 als Wirkstoff und gegebenenfalls übliche pharmazeutische Zusatz-, Hilfs- oder/und Trägermittel.

4. Verwendung eines G-CSF oder eines G-CSF-Derivats nach Anspruch 1 oder 2 zur Herstellung eines Arzneimittels oder von pharmazeutischen Präparaten gegebenenfalls unter Zusatz von üblichen pharmazeutischen Zusatz-, Hilfs-, Füll- oder/und Trägermitteln.

## Revendications (Revendications pour l'(les) Etat(s) contractant(s) suivant(s): DE, FR, GB, IT, AT, BE, LU, NL, SE, DK, CH, LI)

1. Recombinant G-CSF ou dérivé G-CSF recombiné constitué de procaryotes
**caractérisé en ce que**
le G-CSF ou le dérivé G-CSF mature non glycolysé avec la séquence d'acides aminés N-terminal est quantitativement exempt d'un G-CSF constitué de procaryotes avec un résidu de méthionine N-terminal et qu'il est contaminé par moins de 0,1 % d'autres protéines.

2. Recombinant G-CSF ou dérivé G-CSF recombiné selon la revendication 1
**caractérisé en ce que**
le G-CSF ou le dérivé G-CSF est contaminé par moins de 10⁻³ % d'autres protéines.

3. Produit pharmaceutique contenant un G-CSF ou un dérivé G-CSF selon l'une des revendications 1 ou 2 en tant que biocatalyseur et éventuellement d'autres additifs, auxiliaires et/ou supports pharmaceutiques.

4. Utilisation d'un G-CSF ou d'un dérivé G-CSF selon l'une des revendications 1 ou 2 pour la fabrication de préparations pharmaceutiques éventuellement avec adjonction d'autres additifs, auxiliaires, agents de remplissage et/ou supports pharmaceutiques.

## Revendications (Revendications pour l'(les) Etat(s) contractant(s) suivant(s): ES, GR)

1. Procédé de préparation d'un recombinant G-CSF ou dérivé G-CSF recombiné constitué de procaryotes charactérisé en ce que le G-CSF ou le dérivé G-CSF mature non glycosylé avec la séquence d'acides aminés N-terminal est quantitativement exempt d'un G-CSF constitué de procaryotes avec un résidu de méthionine N-terminal et qu'il est contaminé par moins de 0,1 % d'autres protéines.

2. Procédé de préparation d'un recombinant G-CSF ou dérivé G-CSF recombiné selon la revendication 1 charactérisé en ce que le G-CSF ou le dérivé G-CSF est contaminé par moins de 10⁻³% d'autres protéines.

3. Procédé de préparation d'un médicament ou des produits pharmaceutiques contenant un G-CSF ou un dérivé G-CSF selon l'une des revendications 1 ou 2 en tant que biocatalyseur et éventuellement d'autres additifs, auxiliaires et/ou supports pharmaceutiques.

4. Utilisation d'un G-CSF ou d'un dérivé G-CSF selon l'une des revendications 1 ou 2 pour la fabrication d'un médicament ou des produits pharmaceutiques éventuellement avece adjonction d'autres additifs, auxiliaires, agents de remplissage et/ou supports pharmaceutiques.
